# EUROPEAN PATENT APPLICATION

(11) **EP 4 497 403 A1**
(43) Date of publication of application: **29.01.2025**
(21) Application number: 24171553.1
(22) Date of filing: 22.04.2024
(51) Int. Cl.: A61B 18/14, A61B 18/00

(54) **A HAND-HELD SURGICAL INSTRUMENT AND INSULATING INSERT FOR A HAND-HELD SURGICAL INSTRUMENT**

(30) Priority: 26.07.2023 US 202363528992 P
(71) Applicant: Olympus Winter & Ibe GmbH, 22045 Hamburg (DE)
(72) Inventor: KNOPF, Christoph, 23617 Stockelsdorf (DE); BROCKMANN, Christian, 21279 Hollenstedt (DE)
(74) Representative: Hoener, Matthias

(57) **Abstract**

The invention provides an insulation insert for a hand-held surgical device by which a human body is protected or not traumatized by the use of the hand-held device. This is achieved in that an insulating insert (23) for a hand-held surgical device is tubular and has coupling means (26) at a proximal end portion (24) for releasably coupling to a distal end (25) of a tubular inner shaft (13) of the hand-held device. A key feature of the invention is that a circumference of the tubular insulating insert (23) includes at least one flexible component (28). This flexible component (28) on the insulating insert (23) can dampen mechanical forces between the urethra and the handheld device during insertion of the same into the human body.

## Description

The invention relates to an insulating insert for a hand-held surgical instrument according to claim 1. Furthermore, the invention relates to a hand-held surgical instrument according to claim 13.

Surgical instruments, in particular resectoscopes, of the type described are used primarily in urology for electrosurgical work. These devices are usually used for resection and vaporization of tissue, e.g. of tissue in the lower urinary tract. For this purpose, the hand-held instrument, in particular the resectoscope, may comprise a longitudinally displaceable electrosurgical passage instrument which, after insertion of the device into the body to be treated, can be pushed out with its distal working end from a distal end of a shaft tube of the hand-held device. In this regard, the electrosurgical penetrating instrument or the electrode instrument has an electrosurgical electrode at a distal end thereof. This electrode may comprise, for example, the form of a loop or button or the like.

For the above applications, a high-frequency electric current is applied to the electrode. It is important to prevent the electrode from coming into electrical contact with the shaft tube, in particular an outer shaft tube or a sheath tube, of the handpiece. In the event of such electrical contact, a short circuit could cause a device defect or lead to unforeseeable damage in the body to be treated. To avoid such short circuits, the handheld devices or resectoscopes typically include a section at their distal end region or portion that is made of an electrically insulating material. This section is usually referred to as an insulating tip or also as an insulating insert. Here, the insulating insert can be attached either to an inner shaft or shaft tube in which an electrode array is guided or to the outer shaft or sheath tube. Since these hand-held devices or the resectoscopes are designed for multiple use and must therefore be sterilized or autoclaved on a regular basis, the insulating insert must meet high requirements for durability and reprocessability. The choice of materials is therefore usually limited to relatively expensive high-performance ceramics, such as silicon nitride. As an alternative to these ceramics, electrically insulating plastics are used at least for *single-use* articles. These plastics are thermostable in particular, so that they do not melt immediately on contact with the electrode.

The aforementioned materials all behave mechanically as rigid. This property is particularly problematic when inserting the instrument into the urethra. As soon as the insulating insert or part of the insulating insert comes into contact with the urethra, the force used to insert the instrument into the body and all other relative movements between the instrument and the body are transmitted to the urethra or mucosa directly opposite this contact area. Small contact areas in particular lead to a large mechanical tension in the mucosa. This can inevitably lead to trauma or damage to the mucosa.

The invention is therefore based on the problem of creating an insulating insert as well as a hand-held surgical instrument by means of which the body is protected or not traumatized by the use of the instrument.

A solution to this problem is described by the features of claim 1. Accordingly, it is provided that an insulating insert for a hand-held surgical device, in particular for a resectoscope, is tubular and has coupling means at a proximal end portion for releasably coupling to a distal end of a tubular shaft of the hand-held device. A key feature of the invention is that a circumference or aperiphery of the tubular insulating insert includes at least one flexible component. This flexible component on the insulating insert can dampen mechanical forces between the urethra and the hand instrument during insertion of the same into the human body. The mechanical force or tension is distributed over a larger contact area with the mucosa and leads to a deformation of the flexible component. As a result, traumatization of the urethra can be avoided or at least reduced.

Preferably, the at least one flexible component is provided at the circumference or the periphery of a distal end portion of the insulating insert. According to this embodiment, the insulating insert directly contacts the interior of the urethra or the mucosa with the flexible component, which allows the force to be distributed more evenly over the contact area. The positioning of the flexible component at the distal end region or the distal end face of the insulating insert is particularly advantageous for the manufacture of the insulating insert. In addition, the force can be absorbed particularly efficiently by this positioning of the flexible component.

In particular, the invention provides that the flexible component is made of a silicone or an elastomer. The insulating insert can be made of a rigid material, in particular a ceramic or a plastic. By this choice of material, the insulating insert fulfills the requirements described above; at the same time, by combining it with the flexible material, the problem described can be solved and the instrument can be moved in the body in a manner that is particularly gentle on the body. When selecting the material for the flexible component, it is important for reusable instruments that it is in particular heat-resistant and can be reprocessed more frequently.

A preferred embodiment of the invention may provide that the flexible component extends in a ring-like manner around the entire circumference of the distal end portion. In this embodiment, the component may be considered a ring-like flexible extension of the insulating insert. The ring-like design of the flexible component can ensure that mechanical forces are absorbed whenever the insulating insert contacts the mucosa, thereby preventing trauma. The orientation of the instrument or the insulation insert relative to the urethra is irrelevant. This offers the surgeon maximum flexibility in his work.

It is further conceivable that the flexible component (28) has a hardness of 10 Shore A to 90 Shore A, preferably 30 Shore A to 70 Shore A. It has been shown that this hardness is particularly suitable for the application described.

Another alternative embodiment of the invention may provide that the flexible component extends over an angular range of 45° to 180°, in particular over an angular range of 90°, on the entire circumference of the distal end region. In particular, if the distal end region of the insulating insert does not lie in a plane but is, for example, beak-shaped, it may be advantageous if only a most distally oriented portion is provided with the flexible component. Depending on the design of the insulating insert, the flexible component can extend over a defined angular range on the distal circumference.

Preferably, it is conceivable that the flexible component is arranged on the outer side or upper side and/or the inner side and/or the front side of the distal end region and/or the flexible component extends on the outer side in the direction of the proximal end. Depending on the design of the handheld surgical device, as well as the intended use, it may be advantageous for the component to extend over different portions of the distal end region of the insulating insert. The further the flexible component extends in the distal direction, the greater the force-absorbing effect. According to the invention, it is conceivable that the flexible component has a length in the axial direction of 0.3 mm to 7.0 mm, preferably 0.5 mm to 2.0 mm.

In particular, it is conceivable that two, three or more flexible components are arranged along the circumference. For applications where there is both a risk of trauma to the urethra and recourse is to be had to a free edge of the distal region of the insulating insert, it may be envisaged that several individual flexible components are arranged along the circumference.

In addition, it may be a particularly preferred embodiment of the invention that the distal end region of the insulating insert has an axial and/or radial or ring-like undercut for improved adhesion of the flexible component. During manufacture of the insulating insert, the component is molded over the distal region. The undercut provides the flexible component with an anchorage strong enough to prevent loosening of the flexible component when radial or axial forces are applied. The undercut creates an increase in the contact area between the flexible component and the insulating insert. This enlargement of the contact area also serves to stabilize the connection between the flexible component and the insulating insert. According to the design or embodiment example of the insulating insert, it may be advantageous to align the undercut axially, i.e. parallel to a longitudinal axis of the insulating insert, or radially, i.e. along the circumference. As an alternative to the undercut, it is also possible for the distal end region of the insulating insert to have holes or notches or other recesses in which the flexible component can become lodged.

Finally, it is also conceivable that the flexible component and a shell surface of at least the distal portion of the insulating insert form a continuous surface. This design of a continuous surface can avoid snagging of the insulating insert within the urethra or difficulties in sufficient reprocessing for reuse of the insulating insert.

A hand-held surgical instrument for solving the aforementioned task has the features of claim 13. In particular, the hand-held surgical instrument may be a resectoscope or the like. The claimed hand-held surgical instrument has an inner shaft or shaft tube and an outer shaft or sheath tube. The sheath tube is disposed about the inner shaft. According to the invention, an insulating insert according to claim 1 is detachably associated with a distal end of the inner shaft. By this arrangement of the insulating insert at the distal end of both the inner shaft and the outer shaft, it can be prevented that an electrosurgical tool or an electrode, respectively, which is led out of the insulating insert through the inner shaft, comes into electrical contact with the metallic formed shaft tubes.

A preferred embodiment of the invention is described in more detail below with reference to the drawing. In this show:
- Fig. 1: a schematic representation of a hand-held surgical instrument, in particular a resectoscope,
- Fig. 2: a perspective view of an insulating insert,
- Fig. 3: a sectional view of the insulating insert according to Fig. 2,
- Fig. 4: a perspective view of a further embodiment of an insulating insert,
- Fig. 5: a sectional view of the insulating insert according to Fig. 4,
- Fig. 6: a perspective view of a further embodiment of an insulating insert,
- Fig. 7: a sectional view of the insulating insert according to Fig. 6, and
- Fig. 8: a sectional view through a distal end region of the insulating insert.

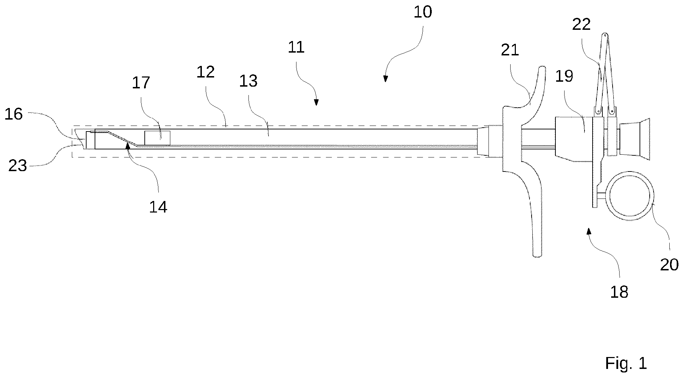

Fig. 1 shows a schematic, lateral sectional view of a resectoscope 10. It should be expressly noted that this resectoscope 10 is shown only as one possible embodiment of the claimed hand-held surgical instrument. Similarly, it is conceivable that the hand-held surgical instrument is an instrument other than the resectoscope 10 shown herein.

The resectoscope 10 has a resectoscope shaft 11 that includes a depicted outer shaft 12 or sheath tube. A tubular inner shaft 13 extends within the outer shaft 12, and an electrode instrument 14 and an indicated optic 15 are shown within the inner shaft 13. In addition, other elements not shown here may extend within the resectoscope 10, such as a separate irrigation tube and the like.

The electrode instrument 14 has an electrosurgical tool or electrode 16 at a distal end. The electrode 16 shown here is shown as a loop, but may also be formed as a button or the like. In Fig. 1, it can be seen that the electrode instrument 14 is protected against displacement, i.e., displacement deviating from the longitudinal direction of the resectoscope shaft 11, by a retaining element 17 having a part-circular cross-section. The electrode instrument 14 is longitudinally displaceably mounted in the inner shaft 13. The holding element 17 has a shape complementary to the inner wall of the inner shaft 13 or to the outer wall of the optics 15 and has a partially cylindrical shape.

The electrode instrument 14 can be forcibly moved axially in the distal and proximal directions by actuating a handle 18. In doing so, it can be pushed beyond the distal end of the inner shaft 13 and the outer shaft 12. This allows the surgeon to manipulate tissue further away from the resectoscope tip. Further, for this purpose, the inner shaft 13 and/or the electrode instrument 14 may be rotatably mounted about their longitudinal axis. For the manipulation of the tissue, a high-frequency electric current is applied to the electrode 16.

The resectoscope 10 shown in Fig. 1 has a passive transporter in which a carriage 19 is displaced in the distal direction against the distal, first handle part 21 by relative movement of the handle parts 20 and 21 arranged proximally on the resectoscope shaft 11 against a spring force applied by a spring bridge 22. When the slide 19 is displaced in the distal direction against the handle part 21, the electrode instrument 14 is displaced in the distal direction in a manner not shown. When the load is released from the handle portions 20, 21, the spring force generated by the spring bridge 22 forces the slide 19 back to its initial position, pulling the electrode instrument 14 in the proximal direction. When the slide 19 is moved back, an electrosurgical procedure can be performed with the electrode 16 without the operator's manual force, i.e. passively.

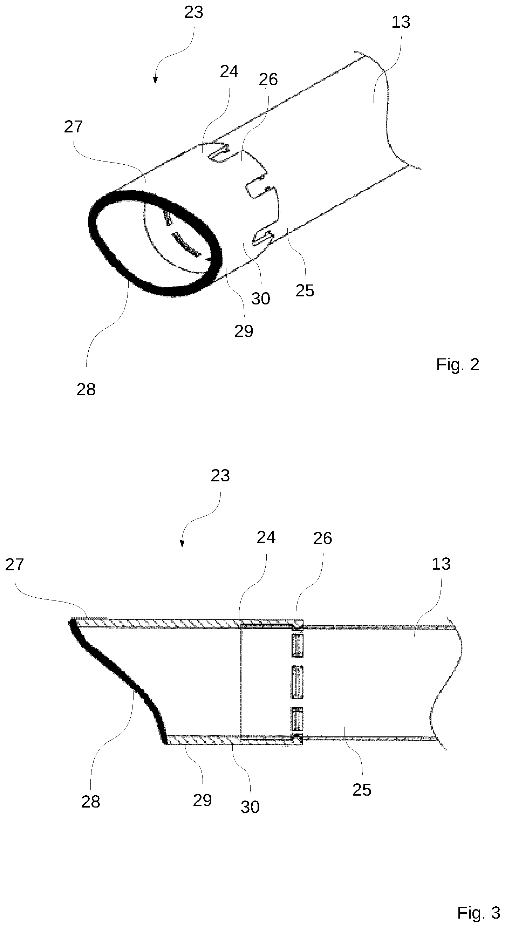

An insulating insert 23 is releasably coupled at its proximal end 24 to the distal end 25 of the inner shaft 13 (see e.g. Fig. 2, only a distal region of the device is shown here). To this end, it is provided that the insulating insert 23 has coupling means 26 at its proximal end 24. To treat the patient, the resectoscope 10 is pushed into the patient's urethra with the insulating insert 23 in front. To prevent the aforementioned traumatization of the urethra or mucosa, a circumference 27 of the insulation insert 23 has a flexible component 28. This flexible component 28 can be associated with the circumference 27 of the distal end region 29 of the insulating insert 23, as shown in the embodiment example according to Figs. 2 and 3. Thereby, in the illustrated embodiment example, the flexible component 28 can be considered as a kind of ring. According to the invention, this flexible component 28 is made of a silicone, an elastomer or another flexible material. If, during insertion of the resectoscope 10, the insulating insert 23 with the flexible component 28 in front encounters the mucosa, there is no injury or traumatization, but rather an elastic deformation of the flexible component 28. Thus, there is no deformation and ultimately injury to the mucosa due to the transmitted mechanical stress. The insulating insert 23 is made of a rigid material, such as a ceramic, a composite, a plastic or the like, so that the shape of the insulating insert 23 does not change per se and the functionality of the resectoscope 10 is not disturbed.

In the example embodiment shown in Figs. 2 and 3, the entire distal end region 29 of the insulating insert 23 is surrounded by the flexible component 28 so that the mucosa of the urethra is protected regardless of the contact area of the insulating insert 23.

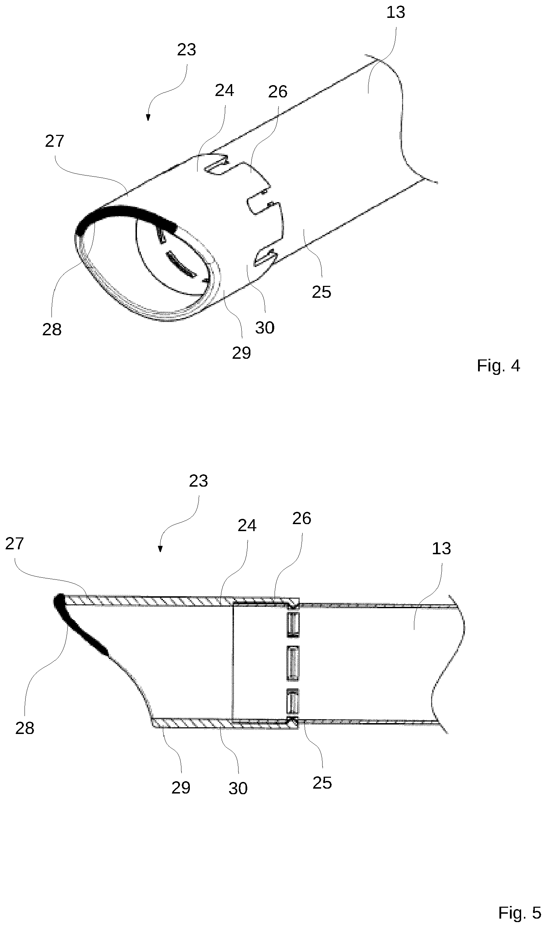

Alternatively, it is equally conceivable that only a partial region of the distal end region 29 of the insulating insert 23 has a flexible component 28. For example, Figs. 4 and 5 illustrate an embodiment in which only an upper region of the periphery or circumference 27 is provided with the flexible component 28. It can be seen that the flexible component 28 extends over the inner side, the front side as well as the outer side of the distal end portion 29. The upper region of the circumference 27 is thus at least partially enclosed by the flexible component 28.

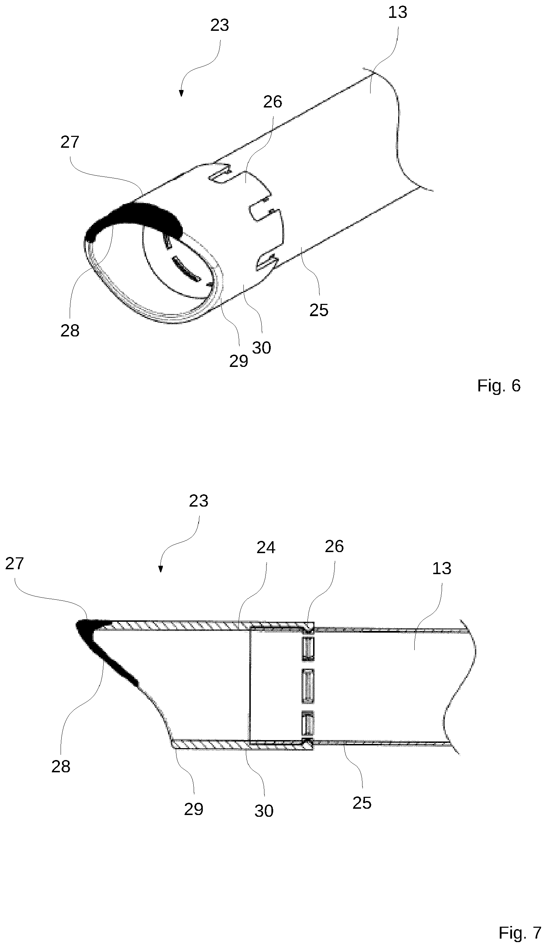

Figs. 6 and 7 show an embodiment in which this enclosing of the upper portion of the circumference 27 is even more pronounced. Here, the flexible component 28 extends on the upper side or on a lateral surface or sheath area 30 of the insulating insert 23 in the direction of the proximal end 24. Due to this enlargement of the flexible component 28, a larger amount of force applied from the outside to the insulating insert 23 or to the mucosa can be absorbed.

According to the invention, it is provided that the flexible component 28 has a width of 0.3 mm to 7.0 mm, preferably 0.5 mm to 2.0 mm. In this case, the component 28 is dimensioned in such a way that it lies in one plane with the sheath area 30 of the insulating insert 23, so that there is no constriction between the outer shaft 12 and the inner shaft 13.

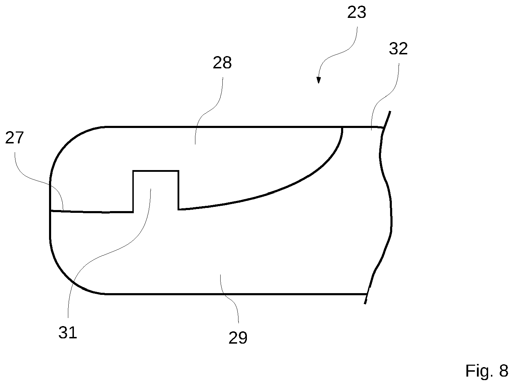

For a particularly stable as well as firm connection between the flexible component 28 and the circumference 27 of the insulating insert 23, it may be provided that the circumference 27 has an undercut 31. As shown, for example, in Fig. 8, the circumference 27 of the distal end portion 29 may be associated with a radial undercut 31 having a cuboid cross-section. This undercut 31 imparts additional mechanical resistance to the flexible component 28, which is injection-molded around the distal end region 29, for example, and protects against unwanted detachment of the flexible component 28 from the insulating insert 23 when an external force is applied. In Fig. 8, only a sectional view of a wall 32 of the insulating insert 23 is shown. Alternatively or additionally, it is also conceivable that an axial undercut or several smaller radial undercuts are associated with the distal end region or portion 29 in order to further increase the stability of the connection between the flexible component 28 and the insulating insert 23.

### List of Reference Numerals:

- 10: Resectoscope
- 11: Resectoscope shaft
- 12: Outer shaft
- 13: Inner shaft
- 14: Electrode instrument
- 15: Optics
- 16: Electrode
- 17: Holding element
- 18: Handle
- 19: Sledge
- 20: Handle part
- 21: Handle part
- 22: Spring bridge
- 23: Insulation insert
- 24: Proximal end
- 25: Distal end
- 26: Coupling means
- 27: Circumference
- 28: Flexible component
- 29: Distal end portion
- 30: Sheath area
- 31: Undercut
- 32: Wall

## Claims

1. Insulating insert (23) for a hand-held surgical device, in particular for a resectoscope (10), said tubular insulating insert (23) having coupling means (26) at a proximal end (24) for releasable coupling to a distal end (25) of a tubular inner shaft (13) of the hand-held device, **characterized in that** a circumference (27) of the tubular insulating insert (23) has at least one flexible component (28).

2. Insulating insert (23) according to claim 1, **characterized in that** the at least one flexible component (28) is arranged at the circumference (27) of a distal end portion (29) of the insulating insert (23).

3. insulating insert (23) according to claim 1 or 2, **characterized in that** the flexible component (28) is made of silicone or an elastomer and/or the insulating insert (23) is made of a rigid material, in particular a ceramic or a plastic.

4. Insulating insert (23) according to any one of the preceding claims, **characterized in that** the flexible component (28) extends annularly around the entire circumference (27) of the distal end portion (29).

5. Insulating insert (23) according to one of the preceding claims, **characterized in that** the flexible component (28) has a hardness of 10 Shore A to 90 Shore A, preferably 30 Shore A to 70 Shore A.

6. Insulating insert (23) according to one of the preceding claims, **characterized in that** the flexible component (28) extends over an angular range of 45° to 180°, in particular over an angular range of 90°, on the entire circumference (27) of the distal end portion (29).

7. Insulating insert (23) according to any one of the preceding claims, **characterized in that** the flexible component (28) is arranged at a portion of the circumference (27) of the distal end portion (29) that extends furthest in the distal direction.

8. insulating insert (23) according to any one of the preceding claims, **characterized in that** the flexible component (28) is arranged on the outer side or upper side and/or the inner side and/or the end side of the distal end portion (29) and/or the flexible component (28) extends on the outer side towards the proximal end (24).

9. Insulating insert (23) according to any one of the preceding claims, **characterized in that** two, three or more flexible components (28) are arranged on the circumference (27).

10. Insulating insert (23) according to any one of the preceding claims, **characterized in that** the flexible component (28) has a length in the axial direction of 0.3 mm to 7.0 mm, preferably 0.5 mm to 2.0 mm.

11. Insulating insert (23) according to one of the preceding claims, **characterized in that** the distal end portion (29) of the insulating insert (23) has an axial and/or radial or ring-like undercut (31) for improved adhesion of the flexible component (28).

12. Insulating insert (23) according to any one of the preceding claims, **characterized in that** the flexible component (28) and a sheath area (30) of at least the distal end portion (29) of the insulating insert (23) form a continuous surface.

13. A hand-held surgical instrument, in particular a resectoscope (10), comprising an inner shaft (13) or shaft tube and an outer shaft (12) or sheath tube, and an insulating insert (23) according to claim 1, wherein the insulating insert (23) is releasably couplable to the inner shaft (13) and the outer shaft (12) is movable over the inner shaft (13) and the insulating insert (23).
